# EUROPEAN PATENT APPLICATION

(11) **EP 0 798 346 A2**
(43) Date of publication of application: **01.10.1997**
(21) Application number: 97301672.8
(22) Date of filing: 12.03.1997
(51) Int. Cl.: C09B 47/04, C09B 67/22, G03G 5/06, C07D 487/22

(54) **Hydroxygallium phthalocyanine and alkoxygallium phthalocyanine dimers**

(30) Priority: 29.03.1996 US 625504
(71) Applicant: XEROX CORPORATION, Rochester New York 14644 (US)
(72) Inventor: Goodman, Donald G., Pittsford, NY 14534 (US); Grammatica, Steven J., Penfield, NY 14526 (US)
(74) Representative: Pike, Christopher Gerard

(57) **Abstract**

A photoconductive imaging member includes a supporting substrate, a photogenerating layer and a charge transport layer, wherein the photogenerating layer comprises a mixture of hydroxymetallo phthalocyanine and alkoxy-bridged galliumophthalocyanine dimers.

## Description

This invention relates to a mixture of hydroxygallium phthalocyanine and alkoxygallium phthalocyanine dimer and an electrophotographic photoreceptor containing the mixture as charge generating material.

Hydroxygallium phthalocyanine is useful as a charge carrier generating pigment in electrophotographic photoreceptors. The hydroxygallium phthalocyanine pigment is important because of its high sensitivity. The present invention relates to a mixture of hydroxygallium phthalocyanine and alkoxygallium phthalocyanine dimer as a charge generating pigment

U.S. Patent No. 5,302,710 to Nukada et al. teaches a phthalocyanine mixed crystal comprising a halogenated indium phthalocyanine and a halogenated gallium phthalocyanine. Nukada et aldiscloses an electrophotographic photoreceptor containing the phthalocyanine mixed crystal. The mixed crystal is prepared by dry milling and treating with an organic solvent Electrophotographic photoreceptors containing the mixed crystal is characterized by improved stability upon repeated use.

U.S. Patent No. 5,418,107 to Nealey et al. teaches a process for fabricating an electrophotographic imaging member that includes a mixture of pigment particles. The mixture comprises two different phthalocyanine pigments free of vanadyl. Nealey et al, teaches typical mixtures including metal-free phthalocyanine and titanyl phthalocyanine particles; chloroindium phthaiocyanine and titanyl phthalocyanine particles; and hydroxygallium phthalocyanine and titanyl phthalocyanine particles.

Allowed copending U.S. Patent Application No. 08/439,395 to Grammatica et al. teaches a blend of TiO phthalocyanine (IV) and chloroindium phthalocyanine to achieve a balanced sensitivity.

In general, phthalocyanine particles or mixtures of phthalocyanine particles are provided for the purposes of improved properties at a particular sensitivity range. Tailoring of the pigments to particular sensitivity ranges provides compositions that have improved properties for particular photoreceptor applications. For example, some compositions can be used as charge generating pigments in photoreceptors that are used in printers having 780 nm laser diode exposure systems. See U.S. Patent Application No.08/439,395 to Grammatica et al.

The present invention is directed to a mixture of crystals that can provide different photoreceptor designs for different applications. The proportions of pigments in the mixture can be varied to provide a range of sensitivities. By changing the proportions of the pigments in the mixture, the mixture can be used in many different applications. A single combination of pigments can be provided in place of separate pigment systems previously used for the different applications.

The present invention provides in one aspect a phthalocyanine pigment comprising a mixture of hydroxygallium phthalocyanine and alkoxygallium phthalocyanine dimer. The present invention provides in another aspect an electrophotographic imaging member comprising the mixture of hydroxygallium phthalocyanine and alkoxygallium phthalocyanine dimer. The present invention provides in a further aspect a pigment that can be tuned to provide different sensitivities for different applications.

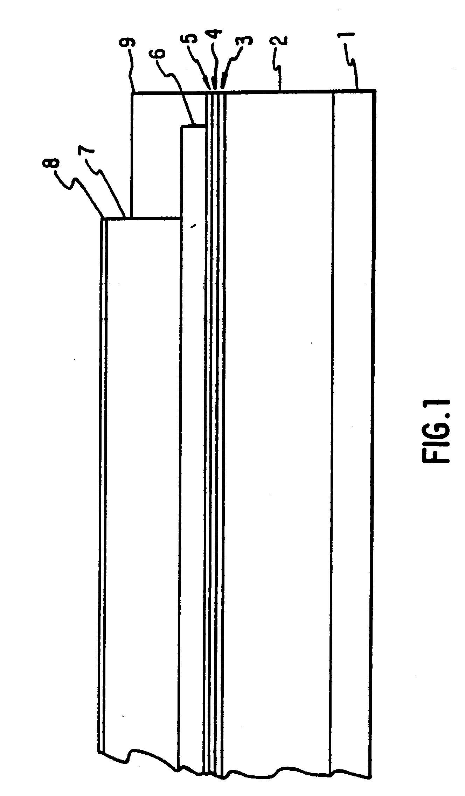

Fig. 1 is a cross-sectional view of a multi-layer photoreceptor of the invention.

Figs. 2-4 are test prints of printings provided by electrophotographic imaging members.

The supporting substrate 2 may be opaque or substantially transparent and may comprise numerous suitable materials having the required mechanical properties. An aluminum drum is the preferred substrate.

The substrate may further be provided with an electrically conductive surface (ground plane 3). Accordingly, the substrate may comprise a layer of an electrically non-conductive or conductive material such as an inorganic or an organic composition. Various known resins can be used as the electrically nonconducting material, including polyesters, polycarbonates, polyamides, polyurethanes, and the like. For a belt-type imaging member, the electrically insulating or conductive substrate should be flexible and may have any number of different configurations such as, for example, a sheet, a scroll, an endless flexible belt, and the like. Preferably, the substrate is in the form of an endless flexible belt and comprises a commercially available biaxially oriented polyester known as Mylar, available from E.I. du Pont de Nemours & Co., or Melinex available from ICI Americas Inc.

The preferred thickness of the substrate layer depends on numerous factors, including economic considerations. The thickness of this layer may range from about 65 micrometers to about 150 micrometers, and preferably from about 75 micrometers to about 125 micrometers for optimum flexibility and minimum induced surface bending stress when cycled around small diameter rollers, e.g., 19 millimeter diameter rollers. The substrate for a flexible belt may be of substantial thickness, for example, 200 micrometers, or of minimum thickness, for example, 200 micrometers, or of minimum thickness, for example 50 micrometers, provided there are no adverse effects on the final photoconductive device. The surface of the substrate layer is preferably cleaned prior to coating to promote greater adhesion of adjacent layer. Cleaning may be effected by exposing the surface of the substrate layer to plasma discharge, ion bombardment and the like.

The electrically conductive ground plane 3 (if needed) may be an electrically conductive layer such as a metal layer which may be formed, for example, on the substrate 2 by any suitable coating technique, such as a vacuum depositing technique. Typical metals include aluminum, zirconium, niobium, tantalum, vanadium, hafnium, titanium, nickel, stainless steel, chromium, tungsten, molybdenum, and the l'ike, and mixtures and alloys thereof. The conductive layer may vary in thickness over substantially wide ranges depending on the optical transparency and flexibility desired for the electrophotoconductive member. Accordingly for a flexible photoresponsive imaging device, the thickness of the conductive layer is preferably between about 2nm to about 75nm, and more preferably from about 5nm to about 20nm for an optimum combination of electrical conductivity, flexibility and light transmission.

Regardless of the technique employed to form a metal layer, a thin layer of metal oxide generally forms on the outer surface of most metals upon exposure to air. Thus, when other layers overlying the metal layer are characterized as "continuous" layers, it is intended that these overlying contiguous layers may, in fact, contact a thin metal oxide layer that has formed on the outer surface of the oxidizable metal layer. Generally for rear erase exposure, a conductive layer light transparency of at lest about 15 percent is desirable. The conductive layer need not be limited to metals. Other examples of conductive layers may be combinations of materials such as conductive 'Indium tin oxide as a transparent layer for light having a wavelength between about 400nm and about 900nm or a conductive carbon black dispersed in a plastic binder as an opaque conductive layer. The conductive ground plane 3 may be omitted if a conductive substrate is used.

After deposition of any electrically conductive ground plane layer, the charge blocking layer 4 may be applied theroto. Electron blocking layers for positively charged photoreceptors allow holes from the imaging surface of the photoreceptor to migrate toward the conductive layer. For negatively charged photoreceptors, any suitable hole blocking layer capable of forming a barrier to prevent hole injection from the conductive layer to the opposite photoconductive layer may be utilized.

The blocking layer 4 may include polymers such as polyvinylbutyral, epoxy resins, polyesters, polysiloxanes, polyamides, polyurethanes and the like; nitrogen-containing siloxanes or nitrogencontaining titanium compounds such as trimethoxysilyl propyl ethylene diamine, beta(aminoethyl) gamma-amino-propyl trimethoxy silane, isopropyl aminobenzene sulfonyl titanate, di(dodecylbenzene sulfonyl) titanate, isopropyl di(4-aminobenzoyl)isostearoyl titanate, isopropyl tri(methylamino) titanate, isopropyl trianthranil titanate, isopropyl tri(N,N dimethylethylamino) titanate, titanium-4-amino benzene sulfonate oxyacetate, titanium 4-aminobenzoate isostearate oxyacetate, [H2N(CH2-)4]CH3Si(OCH3)2 (gamma-aminobutyl methyl dimethoxy silane), [H2N(CH2)3]CH3Si(OCH3)2 (gamma-aminopropyl methyl dimethoxy silane), and [H2N(CH2)3]Si(OCH3)3 (gamma-aminopropyl trimethoxy silane) as disclosed in U.S. Patents Nos. 4,338,387, 4,286,033 and 4,291,1 10.

A preferred hole blocking layer comprises a reaction product of a hydrolyzed silane or mixture of hydrolyzed silanes and the oxidized surface of a metal ground plane layer. The oxidized surface inherently forms on the outer surface of most metal ground plane layers when exposed to air after deposition. This combination enhances electrical stability at low relative humidity. The hydrolyzed silanes that can be used are hydrolyzed silanes that are well known in the art. For example, see U.S. Patent No. 5,091,278 to Teuscher et al.

The blocking layer 4 should be continuous and may have a thickness of up to 2 micrometers depending on the type of material used. A blocking layer of between about 0.005 micrometer and about 0.3 micrometer is satisfactory because charge neutralization after the exposure step is facilitated and good electrical performance is achieved. A thickness between about 0.03 micrometer and about 0.06 micrometer is preferred for blocking layers for optimum electrical behavior.

The blocking layer 4 may be applied by any suitable technique such as spraying, dip coating, draw bar coating, gravure coating, silk screening, air knife coating, reverse roll coating, vacuum deposition, chemical treatment and the like. For convenience in obtaining thin layers, the blocking layer is preferably applied in the form of a dilute solution, with the solvent being removed after deposition of the coating by conventional techniques such as by vacuum, heating and the like. Generally, a weight ratio of blocking layer material and solvent of between about 0.5: 100 to about 5.0: 1 00 is satisfactory for spray coating.

An intermediate layer 5 between the blocking layer and the charge generating or photogenerating layer may be provided to promote adhesion. However in the present invention, a dip coated aluminum drum is the preferred substrate and is utilized without an adhesive layer. When an adhesive layer is utilized, it can be characterized by a dry thickness between about 0.01 micrometer to about 0.3 micrometer, more preferably about 0.05 to about 0.2 micrometer.

An adhesive layer, if utilized, may comprise any known adhesive for layers of an electrophotographic imaging member. The adhesive layer may comprise a film-forming polyester resin adhesive such as du Pont 49,000 resin (available from E.I. du Pont de Nemours & Co.), Vitel 1200 (available from Goodyear Rubber & Tire Co.).

Both the Dupont 49,000 and Vitel 1200 adhesive layers provide reasonable adhesion strength and produce no deleterious electrophotographic impact on the resulting imaging member.

Another copolyester resin adhesive is available from Goodyear Tire & Rubber Co. as Vitel 2200. This polyester resin is a linear saturated copolyester of two diacids and two diols. The molecular structure of this linear saturated copelyester is represented by the following: 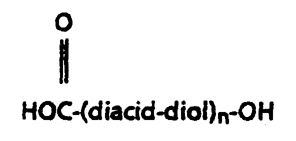 where the ratio of diacid to ethylene glycol in the copelyester is 1: 1. The diacids are terephthalic acid and isophthalic acid in a ratio of 1.2: 1. The two diols are ethylene glycol and 2,2-dimethyl propane diol in a ratio of 1.33:I.The Goodyear Vitel 2200 linear saturated copelyester consists of randomly altemating monomer units of the two diacids and the two diols and has a weight average molecular weight of about 58,000 and a Tg of about 67°C.

Other suitable copolyesters include Goodyear Vitel 1710, Vitel 1870, Vitel 3300, Vitel 3550 and Vitel 5833. Vitel 5833 is a short chained branched polymer having cross-linkable hydroxyl and carboxylic acid functional groups. Vitel 5833 is particularly useful by itself or blended with other polyesters in applications requiring an increase of adhesive layer cross-linking density.

The charge generating layer 6 comprises a polymer binder and a mixture of photoconductive pigments. The photoconductive pigments are a mixture of hydroxygallium phthalocyanine and alkoxygallium phthalocyanine dimer.

In a process for the preparation of hydroxygallium phthalocyanine Type V, pigment precursor Type I chlorogallium phthalocynine is prepared by reaction of about 10 parts to about 100, preferably about 19 parts, gallium chloride in a solvent such as N-methylpyrrolidone, with 1,3diminoisoindolene (DI³) in an amount of about 1 part to about 10 parts, preferably about 4 parts, for each part of gallium chloride. The resulting pigment precursor chlorogailium phthalocyanine is hydrolyzed by standard methods. For example the pigment precursor can be hydrolyzed by acid pasting, wherein the precursor is dissolved in concentrated sulf uric acid and then precipitated in a solvent, such as water, or from a dilute ammonia solution, for example, from about 10 to about 15 percent ammonia. The resulting hydrolyzed hydroxygallium phthalocyanine pigment is treated in a solvent, such as N,N-dimethylformamide, present an amount of from about 1 volume part to about 50 volume parts and preferably about 15 volume parts for each weight of hydroxygallium phthalocyanine. The pigment in solvent is treated by ball milling in the presence of spherical glass beads, approximately 1 millimeter to 5 millimeters in diameter, at room temperature, about 25°C, for a period of from about 1 hours to about 1 week, preferably for about 24 hours. The treatment produces a hydroxygallium phthalocyanine Type V containing very low levels of residual chlorineof from abou to 0.001 percent to about O.1 percent

Additionally, processes for the preparation of hydroxygallium phthalocyanine are illustrated in U.S. Patents No. 5,456,998 to Burt et al., No. 5,466,796 to Burt et al. and No. 5,493,016 to Burt et al.

Additionally, U.S. Patent No. 5,493,016 to Burt et al., U.S. Patent No. 5,466,796 to Burt et al., U.S. Patent No. 5,456,998 to Burt et al. and US Patent No. 5,521,306 to Burt et al. disclose alkoxymetallo phthalocyanine dimers and their preparations that aresuitable in the present invention. U.S. Patent No. 5,493,016 to Burt et al. teaches a process for the preparation of alkoxy-bridged metallophthalocyanine dimers by the reaction of a trivalent metal compound with ortho-phthalodinitrile or 1,3-diminolsoindolene in the presence of a diol. U.S. Patent No. 5,466,796 teaches alkoxy-bridged metallophthalocyanine C₃₂H₁₆N₈MOROMN₈H₁₆C₃₂, wherein M is a metal and R is an alkyl or an alkyl ether. U.S. Patent No. 5,456,998 to Burt et al. teaches photoconductive imaging members comprised of an alkoxybridged metallophthalocyanine dimer as a charge generator material, wherein the dimer is of the formula C₃₂H₁₆N₈MOROMN₈H₁₆C₃₂, where M is a trivalent metal and R is an alkyl group or an alkyl ether group. U.S. Patent No. 5,521,306 to Burt et al. teaches a process for preparation of Type V hydroxygallium phthalocyanine, which comprises in situ forming an alkoxy-bridged gallium phthalocyanine dimer, hydrolyzing the alkoxy-bridged gallium phthalocyanine dimer to hydroxy phthalocyanine and converting the hydroxygallium phthalocyanine product to Type V hydroxygallium phthalocyanine.

Suitable hydroxygallium phthalocyanines and suitable alkoxygallium phthalocyanines are disclosed in US Patent No. 5,521,306 to Burt et al. which in particular discloses alkoxygallium phthalocyanine dimers of the general formula C₃₂H₁₆N₈GaOROGaN₈H₁₆C₃₂ as illustrated by Formula 1: 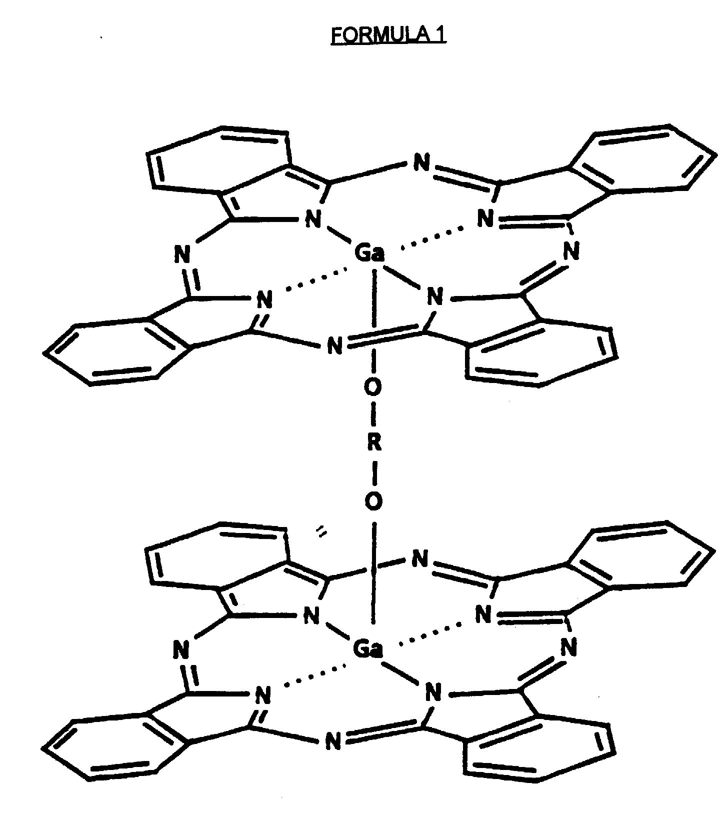 with, for example, from 2 to about 10, and preferably about 2 to 6 carbon atoms in the alkoxy-bridging unit (O-R-O), wherein R is an alkyl group or an alkyl ether.

The charge generating layer is formed by coating on a conductive substrate, a coating composition prepared by dispersing the hydroxygallium phthalocyanine and alkoxygallium phthalocyanine dimer of the present invention in a solution of binder resin in an organic solvent The ratio of the hydroxygallium phthalocyanine to alkoxygallium phthalocyanine dimer as the pigment depends upon the fine tuning required for an application. In general, weight ratios from 99:1 to 1:99 may be used. Preferably, the ratio can be 80:20 to 20:80. Other suitable ratios include 60:40 to 40:60 and 55:45 to 45:55.

A compounding ratio of the phthalocyanine mixture to the binder resin generally ranges from 40/1 to 1/10, and preferably from 10/1 to /1:4, by weight If the ratio of the phthalocyanine mixture is too high, the stability of the coating composition tends to be reduced. If it is too low, the sensitivity of the charge generating layer tends to be reduced.

The solvents to be used in the coating compositions are preferably selected from those incapable of dissolving the lower layer, i.e., the layer on which the charge generating layer is applied. Examples of the organic solvents include alcohols, e.g., methanol, ethanol, and isopropanol; ketones, e.g., acetone, methyl ethyl ketone, and cyclohexanone; amides, e.g., N,N-dimethyiformamide and N,N-dimethylacetamide; dimethyl sulfoxides; ethers, e.g., tetrahydrofuran, dioxane, and ethylene glycol monomethyl ether; esters, e.g., methyl acetate and ethyl acetate; halogenated aliphatic hydrocarbons, e.g., chloroform, methylene chloride, dichloroethylene, carbon tetrachloride, and trichloroethylene; and aromatic hydrocarbons, e.g., benzene, toluene, xylene, ligroin, monochlorobenzene, and dichlorobenzene.

The coating composition for a charge generating layer can be applied by any known coating technique, such as dip coating, spray coating, spin coating, bead coating, wire bar coating, blade coating, roller coating, and curtain coating. Drying after coating is preferably carried out by first drying at room temperatures to the touch and then heat-drying. Heat drying may be performed at a temperature of from 20° to 200°C for a period of from 5 minutes to 2 hours in still air or in an air flow. The charge generating layer usually has a thickness of from about 0.05 to 5 micrometers.

The charge transport layer 7 may comprise any suitable transparent organic polymer or non-polymeric material capable of supporting the injection of photogenerated holes or electrons from the charge generating layer 6 and allowing the transport of these holes or electrons to selectively discharge the surface charge. The charge transport layer not only serves to transport holes or electrons, but also protects the charge generating layer from abrasion or chemical attack and therefore extends the operating life of the imaging member.

The charge transport layer is substantially transparent to radiation in a region in which the imaging member is to be used. The charge transport layer is normally transparent when exposure is effected therethrough to ensure that most of the incident radiation is utilized by the underlying charge generating layer. When used with a transparent substrate, imagewise exposure or erase may be accompl@ished through the substrate with all light passing through the substrate. In this case, the charge transport material need not transmit light in the wavelength region of use.

The charge transport layer may comprise activating compounds dispersed in normally electrically inactive polymeric materials for making these materials electrically active. These compounds may be added to polymeric materials that are incapable of supporting the injection of photogenerated charge and incapable of allowing the transport of this charge. An especially preferred transport layer employed in multilayer photoconductors comprises from about 25 percent to about 75 percent by weight of at least one charge transporting aromatic amine compound, and about 75 percent to about 25 percent by weight of a polymeric film forming resin in which the aromatic amine is soluble.

The charge transport layer is preferably formed from a mixture comprising one or more compounds having the general formula: 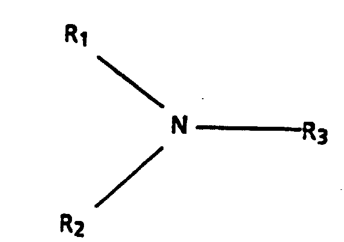 wherein RI and R2 are selected from the group consisting of substituted or unsubstituted phenyl groups, naphthyl groups, and polyphenyl groups and R3 is selected from the group consisting of substituted or unsubstituted aryl groups, alkyl groups having from 1 to 18 carbon atoms and cycloaliphatic groups having from 3 to 18 carbons atoms. The substituents should be free from electron-withdrawing groups such as NO₂ groups, Cₙ groups, and the like.

Examples of charge transporting aromatic amines represented by the structural formula above include triphenylmethane, bis(4diethylamine-2-methylphenyl)- phenylmethane; 4,4'-bis(diethylamino)2,2'-dimethyltriphenylmethane; N,N'-bis(alkyl-phenyl) (1,1'- biphenyl)4,4'-diamine wherein the alkyl is, for example methyl, ethyl, propyl, n-butyl, etc., N,N'-diphenyl-N,N'- bis(3-methylphenyl)-(1,1'biphenyl)-4,4'-diamine; and the like, dispersed in an inactive resin binder.

Any suitable inactive resin binder soluble in methylene chloride or other suitable solvent may be employed. Typical inactive resin binders soluble in methylene chloride include polycarbonate resin, polyvinylcarbazole, polyester, polyacrylate, polyether, polysulfone, and the like. Molecular weights can vary from about 20,000 to 1,500,000. Other solvents that may dissolve these binders include tetrahydrofuran, toluene, trichloroethylene, 1,1,2-trichloroethane, 1,1,1- trichloroethane, and the like.

The preferred electrically inactive resin materials are polycarbonate resins having a molecular weight from about 20,000 to about 120,000, more preferably from about 50,000 to about 100,000. The materials most preferred as the electrically inactive resin materials are poly(4,4'-dipropylidene-diphenylene carbonate) with a molecular weight of from about 35,000 to about 40,000, available as Lexan 145 from General Electric Company; poly(4,4'-isopropylidene-diphenylene carbonate) with a molecular weight of from about 40,000 to about 45,000, available as Lexan 141 from General Electric Company; a polycarbonate resin having a molecular weight of from about 50,000 to about 100,000, available as Makrolon from Farbenfabricken Bayer AG.; a polycarbonate resin having a molecular weight of from about 20,000 to about 50,000, available as Merion from Mobay Chemical Company; polyether carbonates; and 4,4'-cyclohexylidene diphenyl polycarbonate. Methylene chloride solvent is a desirable component of the charge transport layer coating mixture for adequate dissolving of all the components and for its low boiling point

The thickness of the charge transport layer may range from about 10 micrometers to about 50 micrometers, and preferably from about 15 micrometers to about 35 micrometers. Optimum thicknesses may range from about 23 micrometers to about 31 micrometers.

Ground strip 9 may comprise a film-forming binder and electrically conductive particles. Cellulose may be used to disperse the conductive particles. Any suitable electrically conductive particles may be used in the electrically conductive ground strip layer 9. The ground strip 9 may comprise materials which include those enumerated in U.S. Patent No. 4,664,995. Typical electrically conductive particles include carbon black, graphite, copper, sliver, gold, nickel, tantalum, chromium, zirconium, vanadium, niobium, indium tin oxide and the like. The electrically conductive particles may have any suitable shape. Typical shapes include irregular, granular, spherical, elliptical, cubic, flake, filament, and the like. Preferably, the electrically conductive particles should have a particle size less than the thickness of the electrically conductive ground strip layer to avoid an electrically conductive ground strip layer having an excessively irregular outer surface. An average particle size of less than about 10 micrometers generally avoids excessive protrusion of the electrically conductive particles at the outer surface of the dried ground strip layer and ensures relatively uniform dispersion of the particles through the matrix of the dried ground strip layer. Concentration of the conductive particles to be used in the ground strip depends on factors such as the conductivity of the specific conductive particles utilized.

The ground strip layer may have a thickness from about 7 micrometers to about 42 micrometers, and preferably from about 14 micrometers to about 27 micrometers.

The anti-curl layer 1 is optional, and may comprise organic polymers or inorganic polymers that are electrically insulating or slightly semi-conductive. The anticurl layer provides flatness andlor abrasion resistance.

Anti-curl layer 1 may be formed at the back side of the substrate 2, opposite to the imaging layers. The anti-curl layer may comprise a filmforming resin and an adhesion promoter polyester additive. Examples of film-forming resins include polyacrylate, polystyrene, poly(4,4'isopropylidene diphenyl carbonate), 4,4'- cyclohexylidene diphenyl polycarbonate, and the like. Typical adhesion promoters used as additives include 49,000 (du Pont), Vitel PE-100, Vitel PE-200, Vitel PE-307 (Goodyear), and the like. Usually from about 1 to about 15 weight percent adhesion promoter is selected for film-forming resin addition. The thickness of the anti-curl layer is about 3 micrometers to about 35 micrometers, and preferably about 14 micrometers.

The anticurl coating may be applied as a solution prepared by dissolving the film forming resin and the adhesion promoter in a solvent such as methylene chloride. The solution is applied to the rear surface of the supporting substrate (the side opposite to the imaging layers) of the photoreceptor device by hand coating or by other methods known in the art. The coating wet film is then dried to producethe anticurl layer 1.

The optional overcoating layer 8 may comprise organic polymers or inorganic polymers that are capable of transporting charge through the overcoat. The overcoating layer may range in thickness from about 2 micrometers to about 8 micrometers, and preferably from about 3 micrometers to about 6 micrometers. An optimum range of thickness is from about 3 micrometers to about 5 micrometers.

The invention will further be illustrated in the following, nonlimiting examples.

### EXAMPLES

Table 1 lists various blend ratios of hydroxygallium phthalocyanine and alkoxygallium phthalocyanine dimer according to the invention. The pigments were dispersed separately and then blended to the desired ratio. The compositions were evaluated for PIDC sensitivity. The sensitivities are reported in the Table. In the Table, ROGaPc is alkylkoxygallium phthalocyanine wherein R is -OCH₂CH₂0- and HOGaPc is hydroxygallium phthalocyanine.

**Table 1**

| Device No. | ROGaPc:HOGaPc ratio | dV/dX v cm²/erg 350V_{DDP} |
|---|---|---|
| 4238704 | 100:0 | 37 |
| 4256702 | 95:5 | 46 |
| 4256704 | 90:10 | 56 |
| 4249705 | 85:15 | 68 |
| 4249707 | 70:30 | 106 |
| 4249711 | 50:50 | 156 |
| 4256706 | 25:75 | 183 |
| 4256709 | 10:90 | 202 |

The following Table 2 shows dV/dX of a Hewlett Packard laserjet IIP printer, an AppleWriter Select 360 printer and a Hewlett Packard Laserjet 4 printer. These three printers were selected because they represent printers that require a low, medium and high sensitivity photoreceptors.The fact that all three prints are similar with regard to darkness demonstrates that the sensitivity of the photoreceptor has been tuned overthe entire relevant range of sensitivities.

**Table 2**

| Machine | dV/dX |
|---|---|
| P-IIP | 58 |
| 90:10 | 56 |
| Apple LW 360 | 145 |
| 50:50 | 156 |
| HP-4 | 224 |
| 10:90 blend | 202 |

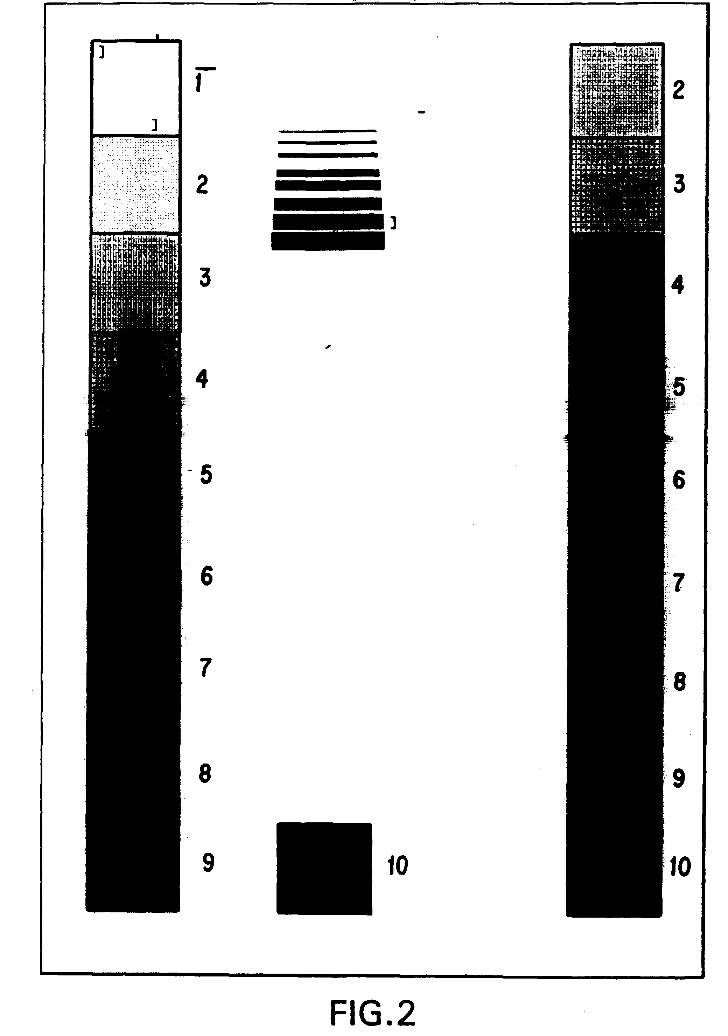

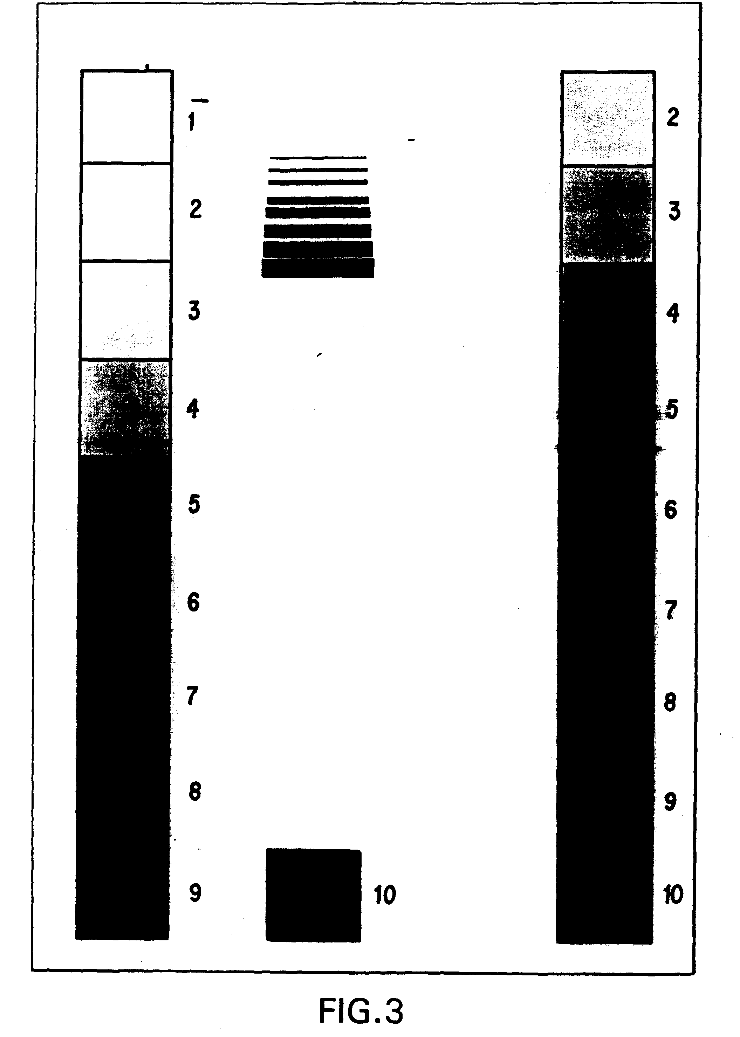

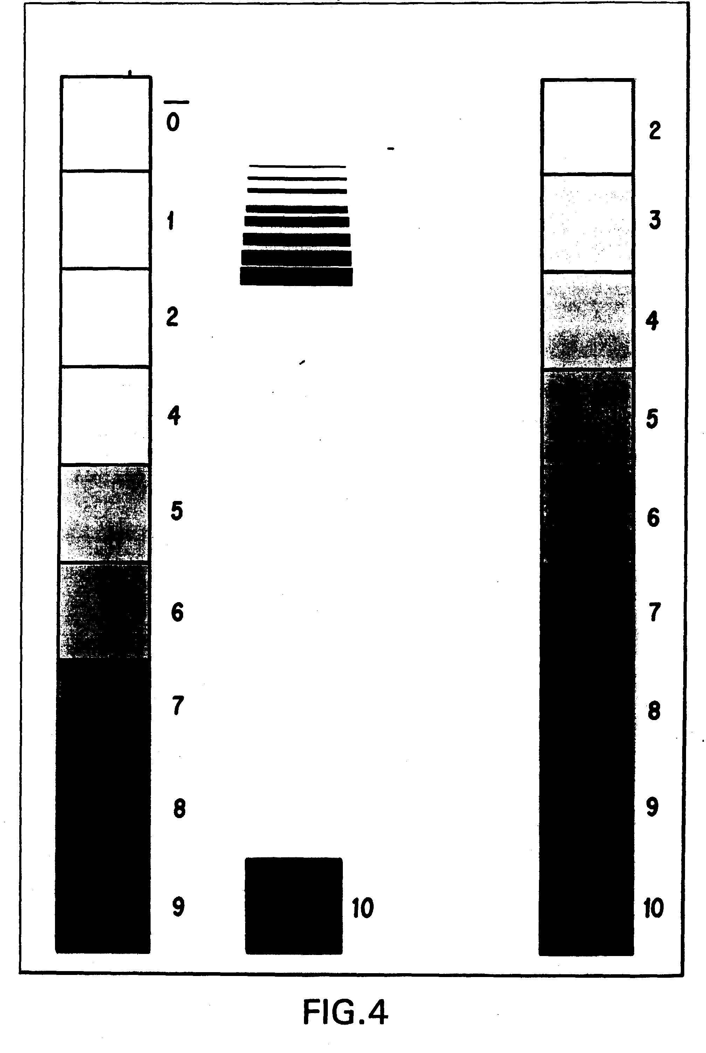

Figure 2 of the drawings shows a test print generated in the Hewlett Packard printer. Figure 3 is a test print generated in a Apple printer. Figure 4 is a test print generated in the Hewlett Packard printer.

The print test results demonstrate that blending mixtures of two pigments in accordance with the invention permits tuning of photoreceptor compositions to provide a range of sensitivities. The tuned photoreceptors are capable of produdng prints comparable to those made using competitive photoreceptors.

The results shown in the Tables illustrate that the present invention provides variable photosensitive compositions. Proportions of hydroxygallium phthalocyanine to alkoxygallium phthalocyanine can be varied as dictated by the sensitivity requirements of a range of applications. The present invention permits compounding of a variety of products by use of only two pigments.

## Claims

1. A phthalocyanine mixture comprising a hydroxygallium phthalocyanine and an alkoxygallium phthalocyanine dimer.

2. The phthalocyanine mixture of claim 1, comprising a weight ratio of said hydroxygallium phthalocyanine to said alkoxygallium phthalocyanine dimer of 99:1 to 1: 99, preferably from 55:45 to 45:55.

3. The phthalocyanine mixture of either of claims 1 or 2, wherein said alkoxygallium phthalocyanine is of the formula: 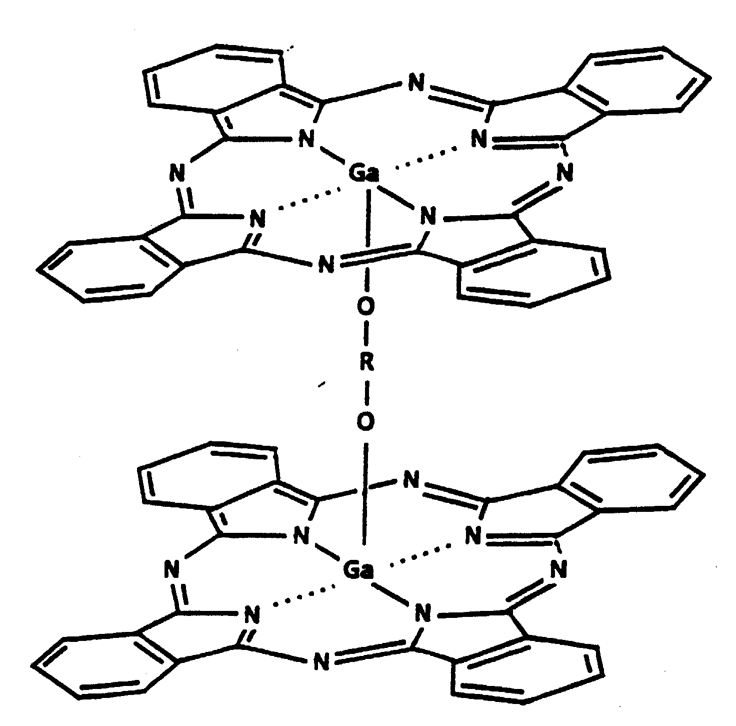 wherein R is an alkyl of 2 to about 1 0 carbon atoms.

4. An electrophotographic member comprising the phthalocyanine mixture of any of claims 1 to 3.

5. An electrophotographic imaging member according to daim 4 comprising said phthalocyanine mixture in a charge generating layer.

6. A method for preparing a photosensitive composition comprising mixing a hydroxygallium phthalocyanine and an alkoxygallium phthalocyanine dimer and forming a photosensitive composition with said mixture.
